Europäisches Patentamt

(19) **European Patent Office**

Office européen des brevets

(11) Publication number: **0 117 016**
**B1**

(12) **EUROPEAN PATENT SPECIFICATION**

(45) Date of publication of patent specification: **08.04.87**

(51) Int. Cl.⁴: **A 61 F 5/44**

(21) Application number: **84300022.5**

(22) Date of filing: **04.01.84**

(54) **Non-return valve arrangement for an ostomy bag or the like.**

(30) Priority: **04.01.83 DK 10/83**

(43) Date of publication of application:
**29.08.84 Bulletin 84/35**

(45) Publication of the grant of the patent:
**08.04.87 Bulletin 87/15**

(84) Designated Contracting States:
**DE FR GB IT SE**

(56) References cited:
**DE-A-2 249 132**
**GB-A-2 058 011**
**US-A-2 796 864**

(73) Proprietor: **COLOPLAST A/S**
**4, Bronzevej**
**DK-3060 Espergaerde (DK)**

(72) Inventor: **Samuelsen, Peter**
**GI. Vallerodvej 40**
**DK-2960 Rungsted Kyst (DK)**

(74) Representative: **Butler, Michael John et al**
**FRANK B. DEHN & CO. Imperial House 15-19**
**Kingsway**
**London, WC2B 6UZ (GB)**

Courier Press, Leamington Spa, England.

## Description

The present invention relates to a non-return valve arrangement for a bag for collecting liquid excretions from the human body, for example an ostomy bag or the like.

Valves of this type are preferably used by patients who have undergone stoma surgery. The excretion from the stoma should be allowed free passage to the reservoir of the bag, but must be prevented from passing backwards into the opening in the body of the patient, where it may cause an infection due to bacterial contamination of the contents of the bag, a deterioration of the general health of the patient resulting. There is known a non-return valve which comprises a valve flap of flexible film or foil and placed between a reservoir portion of the bag and a portion having coupling means to be connected with corresponding fastening means placed around an opening in the body. In one case, the flap may rest against a foil wall and have an opening facing downwards. Alternatively, two flaps may be placed one upon the other, said flaps also being provided with an opening facing downwards. Under normal conditions the flaps are able to ensure a total closure and are also simple and economical to produce, which makes it possible to discard the bag after a relatively short period of use in order to reduce the risk of contamination. However, the bag, which is being carried under the clothing of the patient may under certain circumstances be bent or folded in such a way that the flap is lifted from its abutment and the reservoir portion may as a result be squeezed, its contents being pressed as a cascade against the non-return valve, which under such condition has proved not to be as leak-free as under laboratory conditions.

There is known from DE—A—2249132 a non-return valve arrangement for a bag for collecting liquid excretions from a body, comprising a valve including a valve flap made of a flexible foil and placed between a reservoir portion of the bag and a portion having coupling means to be connected with corresponding fastening means placed around an opening in the body, the valve arrangement further comprising a second valve arranged downstream with respect to the first valve in the direction of flow of liquid excretions, which second valve comprises a flexible foil flap with a portion extending back beyond the opening of the first valve to define a space between said portion and said valve flap of the first valve.

However such an arrangement of two valves of the same type in series is still liable to fail under circumstances where the flaps are not substantially plane.

It is an object of the invention to improve the non-return valve arrangement in order to substantially reduce the risk of infection, the arrangement still being simple and economical to produce and still providing a free passage for excretions.

Thus the arrangement of the present invention is characterised in that the space is in the form of a passage communicating with the reservoir portion of the bag in a region back beyond the opening of the first valve.

The solution proposed in accordance with the invention comprises in reality two valves in series, but the downstream one is provided with a bypass allowing a possible wave of liquid to enter into the chamber between the two valves. As the downstream valve is a non-return valve, the major portion of the liquid leaking into the intermediate chamber will enter through the bypass passage, which is placed alongside the upstream valve and extends in its flow direction. The upstream valve therefore gets very favourable working conditions, when the demands for tightness are strongest. Practical tests have proved the truth of the theory, and the arrangement ensures greater tightness than a bag with a single valve flap or with two valve flaps in series.

Preferably the foil portion of the second valve flap is folded, the edge of the folded portion being level with the lower edge of the first valve flap. This ensures improved flow through the bypass passage, the working conditions of the upstream valve being further improved.

According to a preferred embodiment of the invention, in which the upstream valve consists of two abutting foil flaps, the downstream valve flaps are arranged in abutment with both flaps of the upstream valve. Thereby, a symmetrical arrangement, in which nearly all pressures from the liquid on the valve flaps will be balanced, is obtained.

Two embodiments of the invention will now be described by way of example and with reference to the accompanying drawings, in which:—

Fig. 1 is a plan view of a collection bag for liquid excretions from the human body, including a non-return valve arrangement in accordance with the invention,

Fig. 2 shows schematically a longitudinal section through the bag according to Fig. 1,

Fig. 3 is a plan view of a collecting bag with a modified non-return valve arrangement, and

Fig. 4 shows schematically the bag according to Fig. 3 in longitudinal section.

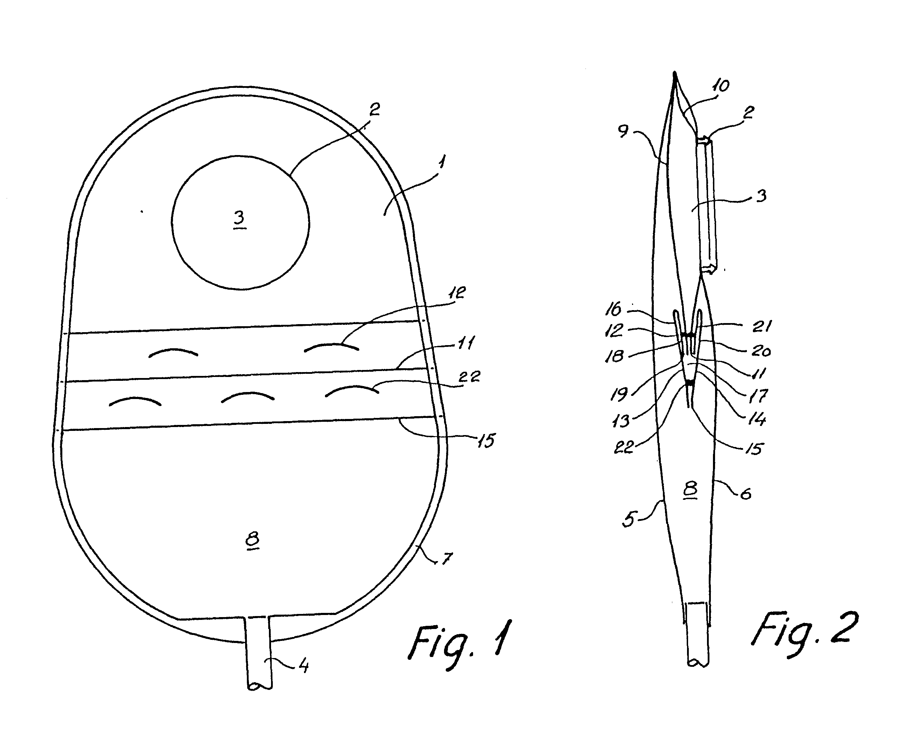

Referring now to Figs. 1 and 2, a non-return valve arrangement is provided in a bag 1, the bag having coupling means 2 for fastening such that an opening 3 within the coupling means is in flow communication with a stoma, fistula or similar opening in a human body. The coupling means enables the bag 1 to be mounted in the body and provided with corresponding coupling means. The bag may be attached direct to the body around the opening, in which case the coupling means is a ring of adhesive intended for attachment to the skin. The bag 1 may also be provided with a drain 4 at the lower, and preferably wider, portion, and the drain may be provided with a closing valve (not shown).

As seen in Fig. 2 the bag comprises two plastic foils 5, 6, one forming the front 5 and the other one forming the back 6. The foils are joined along their edges by means of a welding 7 (Fig. 1). The space between the two foils forms a reservoir portion 8 of the bag. The reservoir portion 8 is separated from the opening 3 by means of a first non-return valve. This valve also comprises two foils, a front foil flap 9 and a rear foil flap 10. The rear foil flap is welded to the back 6 of the bag in the region of the coupling means 2, and the two foil flaps are welded together with the front and back foil in the welding along the edge. The two foil flaps 9 and 10 extend beyond the opening 3, but are cut off at a distance below the opening, with their lower edges 11 at the same level. A short distance above the lower edges 11, the two foil flaps are welded together in small areas, for example along lines 12. Excretions from the opening 3 may pass freely through the space between the foil flaps 9 and 10 into the reservoir portion 8 of the bag 1. If the bag 1 is being hit or squeezed, the foil flaps will be pressed against each other, thereby resisting return of the liquid into the opening in the body.

However, such a simple valve is not sufficiently tight under all circumstances. If the bag is subject to pushing or squeezing and simultaneous bending, a pressure wave in the liquid may result in a leakage through the valve. Therefore, around the first non-return valve a second non-return valve is arranged. This second valve comprises two valve flaps 13, 14, the lower edges 15 of which form a second non-return valve placed in series with the first one. Liquid introuced into the bag may freely pass the two non-return valves into the reservoir portion 8 of the bag. The valve is however, provided with a foil portion 16 passing the lower edge 11 of the upper non-return valve. This foil portion is welded together with the flap 9 of the upper valve along the lines 12. As the weldings are not continuous a bypass passage is formed between the foil portion 16 and the valve flap 9 through which liquid from the reservoir portion may enter into the chamber 17 between the two flaps 13, 14. Liquid entering into the chamber is, however, moving in a direction opposite to a possible leakage through the upper non-return valve and will promote a pressing together of the two foil flaps 9, 10, thereby improving the functioning of the upper non-return valve. In order to reduce the possibility of the foil portion 16 itself functioning as a non-return valve, the foil portion 16 may have a fold 18 facing inwards against the valve flap 9, and the edge 14 of the fold may be in line with the edge 11 of the valve flap 9. Correspondingly the other valve flap 14 may comprise a foil portion 20 with a fold 21. The valve flaps 13, 14 are welded together with the outer foils along the edge and further welded together at lines 22.

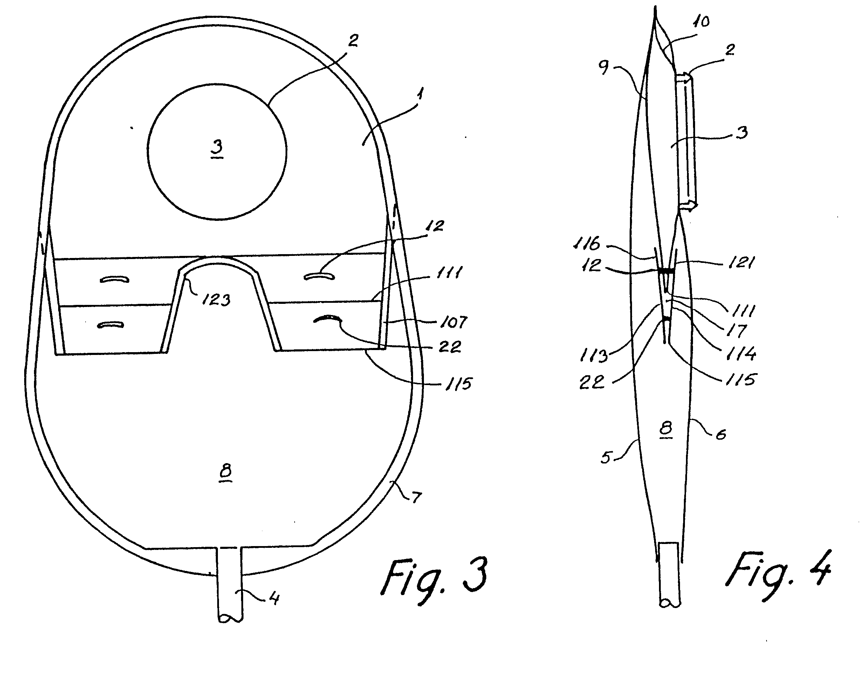

In Fig. 3 and 4 a modified valve arrangement is shown. The bag corresponds in its outer shape and coupling means to the bag according to Fig. 1, and corresponding parts bear the same reference numerals as in Fig. 1 and 2. According to this modification, the foil flaps 9, 10 are not welded into the joint between the front 5 and the back 6 from the level of the coupling means 2, but the two flaps are just welded together along the side edges 107 below this level. In order to reduce the length of the lower edge 111 of the flaps 1, 10, a cut 123 has been made at the centre of the flaps, and the flaps have been welded together along this cut. The flaps of the upper valve are provided with a second valve similar to that described above, having two foil flaps 113 and 114 with lower edges 115 forming a non-return valve. As seen from Fig. 4, the foil portions 116 and 121 are not folded as shown in Fig. 2, but between the flaps 9 and 10 and the foil portions 116 and 121, respectively, a flow passage is defined as described above.

Within the scope of the invention it is possible to simplify the non-return valve arrangement, for example by arranging one or each valve as a single flap being pressed against a continuous foil wall. As an example of such a simplification, with reference to Fig. 2 the valve flap 10 may be extended to the level of the edge 15 or both the valve flaps 10 and 14 can be omitted, the back foil 6 being used as a continuous foil wall. In this case the valve flaps 9 and 13 are welded to the foil 6 at positions corresponding to welds 12 and 22.

**Claims**

1. A non-return valve arrangement for a bag (1) for collecting liquid excretions from a body, comprising a valve (9, 10) including a valve flap (9) made of a flexible foil and placed between a reservoir portion (8) of the bag and a portion having coupling means (2) to be connected with corresponding fastening means placed around an opening in the body, the valve arrangement further comprising a second valve (13, 14) arranged downstream with respect to the first valve (9, 10) in the direction of flow of liquid excretions, which second valve comprises a flexible foil flap (13) with a portion (16) extending back beyond the opening (11) of the first valve to define a space between said portion and said valve flap of the first valve, characterised in that the space is in the form of a passage communicating with the reservoir portion (8) of the bag (1) in a region back beyond the opening (11) of the first valve.

2. An arrangement as claimed in claim 1, characterised in that the foil portion (16) of the second valve flap (13) is folded, the edge (19) of the folded portion being level with the lower edge (11) of the first valve flap (9).

3. An arrangement as claimed in claim 1 or 2, characterised in that the first valve includes two foil flaps (9, 10) abutting each other, and in that on both foil flaps (9, 10) a second foil flap (13, 14) is arranged.

**Patentansprüche**

1. Einwegventilanordnung für einen Beutel (1) zum Sammeln flüssiger Exkretionen von einem Körper, umfassend ein Ventil (9, 10), das eine Ventilklappe (9) enthält, die aus einer flexiblen Folie hergestellt ist und zwischen einem Sammelbehälterabschnitt (8) des Beutels und einem Abschnitt angeordnet ist, der mit entsprechenden, um eine Öffnung im Körper angeordneten Befestigungseinrichtungen zu verbindende Kupplungseinrichtungen (2) besitzt, wobei die Ventilanordnung weiter eine zweites Ventil (13, 14) umfaßt, das in bezug auf das erste Ventil (9, 10) stromabwärts in der Strömungsrichtung von flüssigen Exkretionen angeordnet ist, welches zweite Ventil eine flexible Folienklappe (13) mit einem Abschnitt (16) umfaßt, der sich zurück über die Öffnung (11) des ersten Ventils hinaus erstreckt, um einen Zwischenraum zwischen dem Abschnitt und der Ventilklappe des ersten Ventils festzulegen, dadurch gekennzeichnet, daß der Zwischenraum in der Form einer Durchlaßpassage ist, die mit dem Sammelbehälterabschnitt (8) des Beutels (1) in einem Bereich rückwärts jenseits der Öffnung (11) des ersten Ventils in Verbindung steht.

2. Anordnung nach Anspruch 1, dadurch gekennzeichnet, daß der Folienabschnitt (16) der zweiten Ventilklappe (13) gefaltet ist, wobei der Rand (19) des gefalteten Abschnitts in gleicher Höhe mit dem unteren Rand (11) der ersten Ventilklappe (9) ist.

3. Anordnung nach Anspruch 1 oder 2, dadurch gekennzeichnet, daß das erste Ventil zwei aneinander grenzende Folienklappen (9, 10) enthält und daß auf beiden Folienklappen (9, 10) eine zweite Folienklappe (13, 14) angeordnet ist.

## Revendications

1. Dispositif de soupape à une voie pour un sac (1) à collecter les excrétions liquides d'un corps, comportant un soupape (9, 10) munie notamment d'un clapet de soupape (3) constitué d'une feuille souple et disposé entre une partie réservoir (8) du sac et une partie comportant des moyens de couplage (2) destinés à être connectés à des moyens d'attache correspondants disposés autour d'une ouverture dans le corps, le dispositif de soupape comportant de plus une seconde soupape (13, 14) placés en aval de la première soupape par rapport au sens d'écoulement des excrétions liquides, cette seconde soupape comportant un clapet (13) en feuille souple dont une partie (16) s'étend en arrière au-delà de l'ouverture (11) de la première soupape pour définir un espace entre ladite partie et ledit clapet de la première soupape, caractérisé en ce que l'espace a la forme d'un passage communiquant avec la partie réservoir (8) du sac (1) dans une région située en arrière au-delà de l'ouverture (11) de la première soupape.

2. Dispositif selon la revendication 1, caractérisé en ce que la partie de feuille (16) du second clapet (13) de soupape est repliée, le bord (19) de la partie repliée étant au niveau du bord inférieur (11) du clapet (9) de la première soupape.

3. Dispositif selon l'une des revendications 1 ou 2, caractérisé en ce que la première soupape comporte notamment deux feuilles formant clapet (9, 10) venant au contact l'une de l'autre et en ce que sur chacune des deux feuilles formant clapet (9, 10) est disposée une seconde feuille formant clapet (13, 14).

Fig. 1

Fig. 2

Fig. 3

Fig. 4